# EUROPEAN PATENT SPECIFICATION

(11) **EP 0 541 091 B1**
(45) Date of publication and mention of the grant of the patent: **29.01.1997**
(21) Application number: 92118977.5
(22) Date of filing: 05.11.1992
(51) Int. Cl.: A61B 10/00

(54) **Urine sampler**
Urinsammelvorrichtung
Dispositif de prélèvement d'urine

(30) Priority: 08.11.1991 JP 319727/91; 05.11.1992 JP 319252/92
(43) Date of publication of application: 12.05.1993
(73) Proprietor: Sakai, Yoshisuke, Fukuoka-shi Fukuoka-pref. (JP)
(72) Inventor: Saito, Koichiro, 2-Chome Minato-ku Tokyo (JP); Shimizu, Kaoru, Kisaimachi Kita-Saitama-gun Saitama-Pref (JP)
(74) Representative: Strasser, Wolfgang, Dipl.-Phys

(56) References cited:
- WO-A-84/03212
- US-A- 3 509 872
- US-A- 3 900 019
- US-A- 4 014 322
- US-A- 4 116 066
- US-A- 4 596 157

## Description

### BACKGROUND OF THE INVENTION

### Field of the Invention

This invention relates to a urine sampler for use in conducting urinalysis at schools, hospitals, test centers and the like.

### Description of the Prior Art

Urinalysis, a test that can provide important information for early detection of diseases of the internal organs and otherwise determining the state of the subject's health, can be conducted with no discomfort to the subject and with minimal trouble to those conducting the test. This has led hospitals and test centers to routinely test the urine of large numbers of new patients, persons having medical checkups, hospitalized patients and others.

Ordinarily, the subject goes to a toilet to collect a urine sample in a paper cup and then either places the cup on a designated shelf in a lavatory or dips a test paper into the urine and places only the wetted test paper at a designated place. After a prescribed number of the paper cups containing the samples have been collected from the designated lavatories etc. and brought to the laboratory, the samples are analyzed.

This conventional method of analyzing urine samples collected in paper cups has a number of drawbacks. One is that some people have difficulty getting the urine into the paper cup. This is particularly true of female and elderly persons, who frequently also get urine on their hands and clothing. The method thus has a sanitation problem. In addition, much care is required in handling the collected samples because there is a danger of tipping over the cup when immersing a test paper in the urine or of spilling urine from the cup when carrying it to the designated place.

When, as is frequently the case, a large number of urine samples are tested throughout the day, the wide-mouthed cups, which have to be placed side by side, take up considerable space so that a fairly large area has to be reserved for storing them until the time of testing. There is also a danger of losing all or part of the contents of some of the cups by dropping or tilting them at the time of moving them to the laboratory. This not only fouls the surroundings but may make it impossible to test some samples. Another problem is that many subjects collect larger samples than necessary. As a result, when the samples of a large number of subjects are brought together, they produce a pungent odor which may distract and lower the working efficiency of the doctors, nurses and other personnel who conduct the urinalysis.

In US-A-3.509.872 a small inert absorbent reagent holder is disclosed which generally has the form of a cylinder which at one of its ends is mounted on the end of a plastic stick serving as a handle. The reagent holder is, for example, impregnated with a test reagent which is sensitive to certain urinary infections so as to turn red on contact therewith. The reagent holder is substantially circular in cross-section and is covered by a cylindrical shield maintained in position on the holder by friction. The shield is provided with a number of perforations for the flow of urine through the shield into contact with the reagent holder.

### SUMMARY OF THE INVENTION

The object of this invention is to provide a compact urine sampler which is extremely easy to use for collecting a urine sample of the minimum required quantity and which enables the collected sample to be safely transferred, without spilling, to a sealed container.

For achieving this object, the invention provides a urine sampler as defined in claim 1.

Preferred embodiments of the inventive urine sampler are defined in claims 2 to 11.

With the urine sampler according to the invention, when urine is passed onto the urine collection apertures of the urine collector, the minimum required amount of urine passes through the urine collection apertures to be stored inside the urine accumulator of the elongate rod-like body. By holding the urine sampler by the long rod-shaped handle and urinating onto the urine collection apertures, the user can easily cause a urine sample of the minimum required volume to be collected through the urine collection apertures into the urine accumulator. Moreover, in the arrangement in which the urine collector and the urine storage capsule are separately constituted, after urine has been collected in the urine accumulator, the accumulated urine can be transferred to the urine storage capsule by forcing the cutter of the urine container onto the bottom of the urine accumulator so as to pierce the bottom of the urine accumulator and allow the urine to flow from the urine accumulator into the urine container. Then once the urine storage capsule has been tightly closed with the lid, the urine storage capsule can be carried or transported standing upright with the collected urine stored therein.

### BRIEF DESCRIPTION OF THE DRAWINGS

Figure 1 is a perspective view of a urine sampling stick.

Figure 2 is a perspective view of the individual components of the urine sampling stick of Figure 1.

Figure 3 is a partial view of the urine sampling stick of Figure 1, for showing the amount of urine collected.

Figure 4 is perspective view of a urine sampling stick holder.

Figure 5 is partial view of the urine sampling stick of Figure 1, for showing how a test paper is inserted.

Figure 6 is perspective view showing another embodiment of the urine sampling stick.

Figure 7 is a front view of an embodiment of a urine collection device.

Figure 8 a sectional view of the urine collector of the urine collection device of Figure 7.

Figure 9 is sectional view of the urine collection device of Figure 7 showing a urine capsule thereof joined with the urine collector.

Figure 10 a plan view showing a cutter and the underside of a lid of the urine storage capsule.

Figure 11 is a sectional view of the urine storage capsule.

### DETAILED DESCRIPTION OF THE PREFERRED EMBODIMENTS

The invention will now be explained with reference to the drawings.

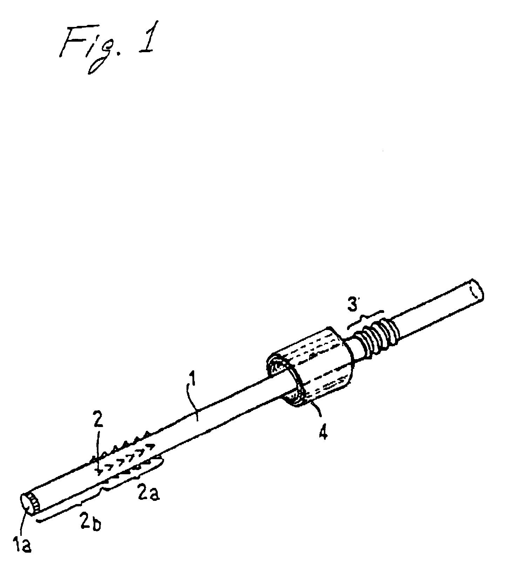

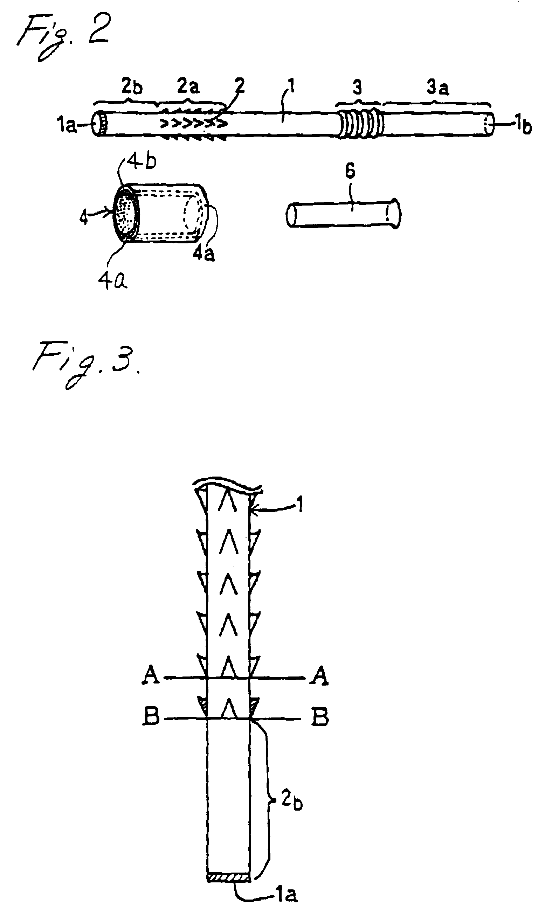

A stick type urine sampler that is an embodiment of this invention is shown in the perspective view of Figure 1. The components of the urine sampling stick are shown in Figure 2. While the stick can be constituted in various sizes, for the purposes of the present embodiment it will be assumed to be 6 mm in diameter and 210 mm in length. The urine sampling stick of this embodiment comprises a main stick body 1, a cover cylinder 4 and a cap 6.

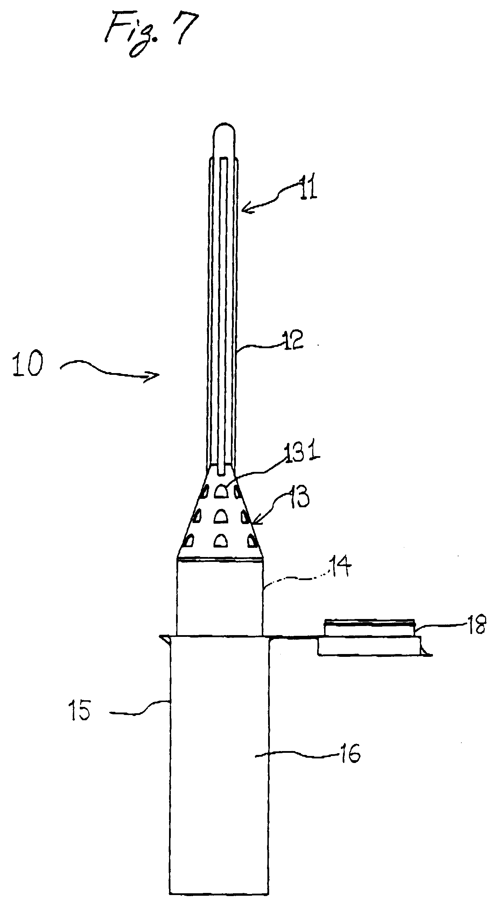

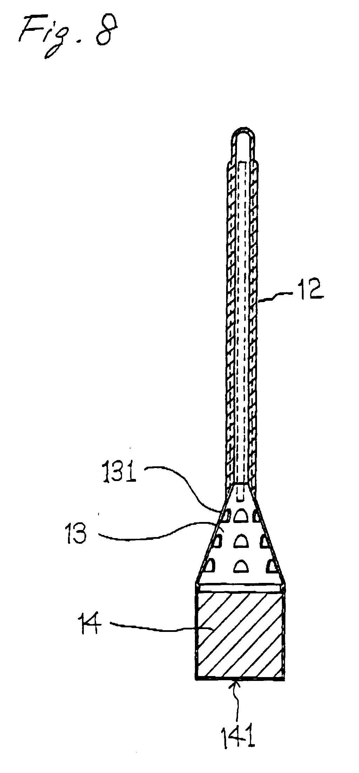

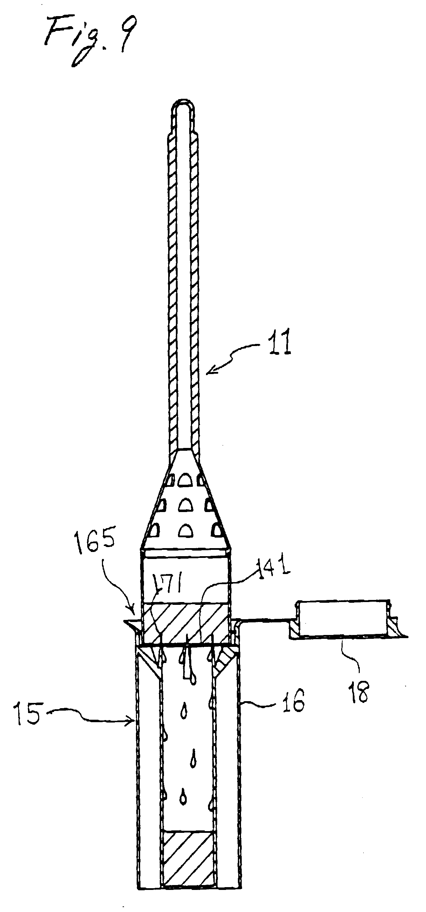

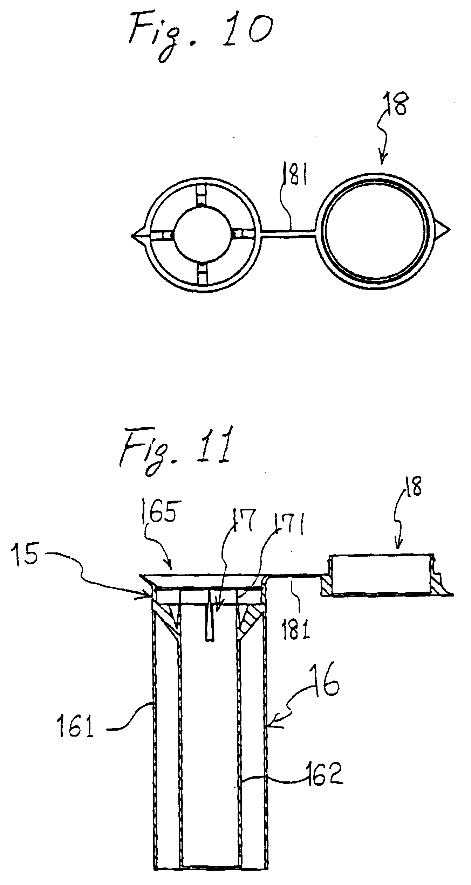

A front view of a urine collection device 10 that is another embodiment of the invention is shown in Figure 7, a sectional view of a urine collector 11 of this embodiment is shown in Figure 8, a sectional view of the urine collection device 10 joined with a urine storage capsule 15 is shown in Figure 9, a plan view of a cutter 17 of the urine storage capsule 15 and the underside of a lid thereof is shown in Figure 10, and a sectional view of the urine storage capsule 15 is shown in Figure 11.

The main stick body 1 of the stick type urine sampler is a colorless transparent or semitransparent straw. The whole urine sampler should be constituted of materials that do not produce noxious gases when burned. One end of the main stick body 1 is closed by a plug 1a and the other end is left open. The closed end will be referred to as the bottom end and the open end as the top end. The wall of the main stick body 1 near the bottom end is formed with a plurality of diagonal cuts 2 to serve as urine collection apertures. The part of the main stick body 1 provided with the cuts 2 constitutes a urine collection section 2a and the part thereof between the urine collection section 2a and the bottom end constitutes a urine storage section 2b. The tips of the cuts 2 project out from the outer surface of the wall of the main stick body 1. The main stick body 1 has a bellows-like corrugated section 3 near its top end.

The cover cylinder 4 has a prescribed length and is fitted on the main stick body 1 to be slidable between the urine collection section 2a and the corrugated section 3. The cover cylinder 4 consists of an outer cylinder portion 4a made of synthetic resin or metal and an inner cylinder portion 4b made of highly water absorbent waste paper or the like.

The cap 6 has an inner diameter enabling it to fit onto the main stick body 1 in snug contact with its outer wall surface and a length that is somewhat greater than the combined length of the urine storage section 2b and the urine collection section 2a. As shown in Figure 2, the open end of the cap 6 is slightly flared to make it easy to fit onto the main stick body 1. To prevent confusion and enable easy identification of the collected urine samples in cases where many samples are tested at the same time, it is preferable to provide the cap 6 (and also the lid 18 explained below in connection with another embodiment) with a place for writing or attaching the name or number of the patient concerned.

As shown in Figure 7, for example, a second embodiment of the urine sampler according to this invention is constituted as a urine collection device 10 consisting of a urine collector 11 and a urine storage capsule 15 that can be joined and separated. The urine collector 11 has a handle 12 and a urine collection member 13 formed integrally therewith, while the urine storage capsule 15 consists of a urine container 16, a cutter 17 and an integral lid 18.

In this embodiment, the urine collection member 13 and the urine accumulator 14 are formed integrally of the same material and the handle 12 is constituted as a long, hollow rod closed at the ends. Since the handle 12 is provided merely as a means for holding the urine collection member 13 and the urine accumulator 14, however, it does not necessarily have to be hollow and is not particularly limited as to material.

The urine collection member 13 is formed to extend integrally from the bottom end of the handle 12 in the shape of a cone. The outer surface of the cone is provided with urine collection apertures 131 which can be formed either by making cuts in the wall of the urine collection member 13 or by making holes therein.

Since the urine collection member flares conically, the subject is able to collect a urine sample in the urine collector simply and reliably, merely by urinating on the urine collecting member 13.

The urine accumulator 14 is a cylinder of the same diameter as the base of the conical urine collection member 13 and is formed integrally with urine collection member 13 to extend downward therefrom. As shown in Figure 8, its bottom 141 is formed as a thin membrane that can be easily pierced by a sharp member (cutter). The bottom 141 can be formed of any material that can be easily pierced by a pointed object, typical examples being thin film of plastic and metal foil.

As shown in the sectional view of Figure 9, the urine container 16 of the urine capsule 15 is a cylinder that is closed at the bottom and has an opening 165 at the top. It is larger in diameter than the urine accumulator 14 so that the bottom of the urine accumulator 14 can be fitted into the top of the urine capsule 15. As shown in the sectional view of Figure 11, urine container 16 consists of an outer cylinder 161 constituting its outer wall and an inner cylinder 162 for holding urine disposed inside and concentrically with the outer cylinder 161. The space between the outer and inner cylinders is left empty.

A cutter 17 for piercing the bottom of the urine accumulator 14 is provided near the top of the inner cylinder 162. The cutter 17 has sharp projections constituting upward pointing blades 171. The precent embodiment is provided with four blades 171 spaced regularly along the upper edge of the inner cylinder 162.

As shown in Figures 10 and 11, the lid 18 has a hinge member 181 by which it is integrally connected with the periphery of the outer cylinder 161 of the urine container 16. The lid 18 is formed to fit into the opening 165 of the urine container 16 snugly enough to prevent the urine contained in the urine container 16 from leaking out even if the urine storage capsule 15 should tip over. While in the present embodiment the snug fit is achieved by a one-touch operation, it is alternatively possible to use a screw-on type lid.

The method in which the stick type urine sampler is used for collecting a urine sample will now be explained along with a method for storing the sticks. The cap 6 is fitted onto the portion of the main stick body 1 above the corrugated section 3 prior to collecting a urine sample. The subject holds the top end of the stick so that the bottom end thereof (the plug 1a end) points sightly downward. Then, after starting to urinate, the subject moves the urine collection section 2a into and out of the urine stream so as to collect only the intermediate portion of the urine passed and not the first and last portions. Urine passed onto the urine collection section 2a flows through the holes formed by the cuts 2 into the interior of the stick and accumulates in the urine storage section 2b. At most, the urine accumulates to the level A-A in Figure 3, at which an equilibrium is reached between the urine pressure and surface tension. However, when the urine adhering to the surface of the stick is wiped off using the cover cylinder 4 or the like, the urine level falls to B-B. In effect, therefore, the amount of urine collected can be considered to be equal to the internal volume of the urine storage section 2b. If the amount of urine required for urinalysis differs depending on the purpose of the test, it suffices to change the length of the urine storage section accordingly.

Since the urine sampler according to this embodiment makes it possible to collect a urine sample merely by bringing the urine collection apertures into the urine stream, it can be easily used by anyone. Moreover, since the distance between the urine collection section 2a and the part of the main stick body 1 that is held with the hand (or in other embodiments, the handle 12) is greater than the splash-back distance of the urine striking the urine storage section 2a (urine collection member 13), any urine which splashes during sampling does not reach the hand holding the urine sampler. The sampling can thus be conducted in a sanitary manner.

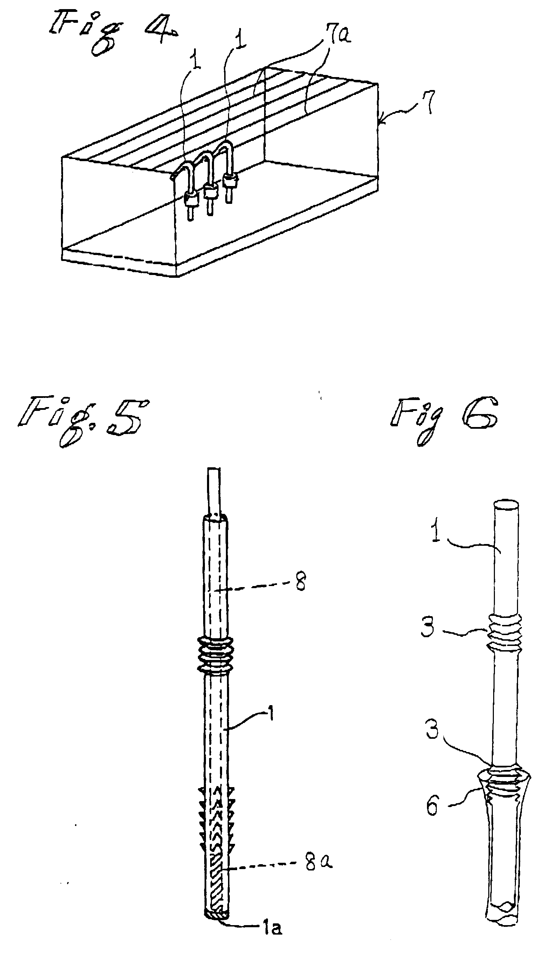

After the urine sample has been taken, the cover cylinder 4, which is initially raised to the bottom edge of the corrugated section 3, is taken between the fingers and lowered to the upper end of the urine collection section 2a so as to wipe off any urine adhering to the surface of the stick. This ensures that urine will not get onto the hands or clothing of the user. Since the cover cylinder 4 has the inner cylinder portion 4b made of highly absorbent waste paper 4b, the urine adhering to the surface of the stick can be thoroughly removed. Although the surface tension inside the stick keeps the urine from flowing out of the urine storage section 2b even when the stick is held horizontally, the urine will flow out if the stick is laid on top of a desk or the like. To prevent this, the cap 6 is pulled off of the top end of the stick and pushed onto the bottom end of the stick until its upper end reaches the upper end of the urine collection section 2a. The urine sampler is then bent at the corrugated section near its upper end and stored, as shown in Figure 4 for example, by hanging it from a wire 7a of a urine sampling stick holder 7 provided in the lavatory. When a number of samples are ready for testing, the urine sampling stick holder 7 is taken to the laboratory. Because of their small size, the urine sampling sticks take up less space in the laboratory than the same number of paper cups. Moreover, owing to the cap 6 fitted on the lower end of the stick and the effect of the internal surface tension, the collected urine does not leak from the stick even if it is tilted or dropped.

The method of conducting the urinalysis will now be explained. The main stick body 1 is held with its top end up as shown in Figure 5, and a urinalysis test paper 8 with several urinalysis reagent patches is inserted from the upper end opening until it reaches the bottom end of the main stick body 1, so as to immerse the reagent section 8a in the urine. The urinalysis reagents thus react with the urine components. The test results are visually observed through the transparent main stick body 1 and recorded. Since there is no need to extract the test paper 8 from the stick, the tester's hands do not come in contact with the urine and the work can be can be conducted in a sanitary manner. Moreover, since only the minimum amount of urine required for testing is collected and this is held in the bottommost part of the stick covered by the cap 6, the tester is exposed to little if any unpleasant smell. After the results of the test have been recorded, the stick is discarded.

Figure 6 shows another embodiment of the urine sampling stick in which the corrugated section 3 is formed in upper and lower segments, the cap 6 fitted snugly onto the main stick body 1 from the lower end thereof is made long enough to reach the lower segment of the corrugated section 3, and the cover 4 of the preceding embodiment is omitted. This arrangement also allows the stick to be handled without getting urine on the hands or clothes and enables the stick to be hung from the wire 7a of the urine sampling stick holder 7, after it has been bent at the upper segment of the corrugated section 3.

The method of collecting a urine sample using the urine collector shown in Figure 8 and the method for transferring the collected sample to a urine storage capsule (the urine collector and the urine storage capsule together constituting another embodiment of this invention) will now be explained. When this embodiment is used for sampling urine, the patient grasps the upper part of the handle 12 of the urine collector 11 (with the urine storage capsule 15 detached), holds the urine collector 11 with the urine collection member 13 and the urine accumulator 14 pointing generally downward, and urinates onto the urine collection member 13. The urine striking the urine collection member 13 passes through the cuts or holes 131 formed in the surface of the urine collection member 13 and passes to the interior to collect in the urine accumulator 14. Preferably, the patient should collect only the intermediate portion of the urine passed and not the first and last portions. Since the urine collection member flares to a larger diameter than that of the handle 12, urine sampling is easier than it would otherwise be. If the amount of urine required for urinalysis differs depending on the purpose of the test, it suffices to change the amount of urine that is collected accordingly.

When the collected urine is to be transferred to the urine storage capsule, the urine collector 11 containing the sampled urine is pressed onto the opening 165 of the urine storage capsule 15 from above. As a result, the sharp blades 171 provided at the top of the urine storage capsule 15 rupture the thin membrane constituting the bottom 141 of the urine accumulator 14, whereby the urine held in the urine accumulator 14 flows into the urine container 16. After all of the sampled urine has passed into the urine container 16, the urine collector 11 is removed from the urine storage capsule 15 and the opening of the urine container 16 is closed and sealed with the lid 18. Later, after the lid has been opened in preparation for urinalysis, the urine can be transferred to a test tube and tested or can be tested by inserting a test paper into the urine while it is still in the urine storage capsule.

The embodiments of the urine sampler described in the foregoing have various advantages:
1. Since a sample can be collected merely by urinating onto the urine collection section/member, anyone is able to use the urine sampler without getting urine on the hands or clothes.
2. Since the sampled urine can be accumulated in the urine storage section/capsule more easily that in can be in the conventional paper cups, the urine sampler is easy and safe to use and also enables the urine to be moved from the lavatory or the like to the laboratory in a highly sanitary manner.
3. The collected samples are easy to store and large numbers of samples can be easily kept in order during storage and testing.
4. Since the urine sampler holds the urine sample in a sealed state that does not allow the odor of the urine to escape, the urinalysis can be conducted in a pleasant working environment without discomfort to those conducting the test.
5. In the case of the urine sampler having the urine storage capsule, the capsule can stand upright on its own, and since it is sealed with a tight lid, there is no danger of the sample spilling or splashing even if the capsule should be knocked off the testing table.

## Claims

1. A urine sampler for being hold into the urine stream from a urinating subject comprising an elongate rod-like body (1; 12) which, near one of its ends, is provided with a hollow urine collector (2a; 13) having a plurality of urine collection apertures (2; 131) in its wall through which apertures (2; 131) urine flows into the interior of said urine collector (2a; 13) and in that it further comprises a hollow urine accumulator (2b; 14) which is integrally connected to said urine collector (2a; 13) so that the interior of said hollow urine accumulator (2b; 14) communicates with the interior of said hollow urine collector (2a; 13), whereby urine collected by said urine collector (2a; 13) further flows into said urine accumulator (2a; 13) where it is stored.

2. A urine sampler according to claim 1, characterized in that the urine collection apertures (2) are formed as cuts by slicing diagonally into the wall of the hollow urine collector (2a) and outwardly raising the tips of the cut portions.

3. A urine sampler according to claim 1 or 2, characterized in that a part of the wall of the rod-like body (1) between the urine collector (2a) and the opposite end is formed into a corrugated section (3).

4. A urine sampler according to claim 3, characterized in that it further is provided with a highly water absorbent cover cylinder (4) fitted onto the rod-like body (1) to be slidable in close contact with the outer surface of the wall thereof between the urine collection apertures (2) and the corrugated section (3).

5. A urine sampler according to any of claims 1 to 4, characterized in that it further comprises a cap (6) snugly fittable onto said urine collector (2a) for closing the urine collection apertures (2).

6. A urine sampler according to claim 5, characterized in that the cap (6) can be forced onto the rod-like body (1) until the open end thereof reaches the corrugated section (3).

7. A urine sampler according to any of claims 1 to 6, characterized in that a test paper (8) can be inserted into the interior of the rod-like body (1).

8. A urine sampler according to any of the preceding claims characterized in that it further comprises a urine storage capsule (15) having a cutter (17) for cutting a portion of the urine accumulator (14), a urine container (16) for receiving urine flowing in from the urine accumulator (14), and a lid (18), the urine accumulator (14) and the urine storage capsule (15) being separately constituted.

9. A urine sampler according to claim 8, characterized in that the rod-like body (12) is a hollow handle closed at the ends, the urine collector (13) flaring downward from the handle in the shape of a cone, and the urine accumulator (14) being a cylindrical body closed at the bottom by an easily pierced thin membrane (141).

10. A urine sampler according to claim 8, characterized in that the cutter (17) of the urine storage capsule (15) is constituted of sharp blades, the urine container (16) is a cylindrical body closed at the bottom, and the lid (18) is formed to fit snugly into the opening (165) of the urine container (16) for preventing leakage of urine thereform.

11. A urine sampler according to claim 9 or 10, characterized in that the urine collection apertures (131) are cuts or holes.

## Patentansprüche

1. Urin-Sammeleinrichtung, die dazu dient, von einer urinierenden Person in den Urinstrahl gehalten zu werden und die einen länglichen, stabartigen Körper (1; 12) umfaßt, der in der Nähe seines einen Endes mit einem hohlen Urinsammler (2a, 13) versehen ist, der eine Vielzahl von Urin-Sammelöffnungen (2; 131) in seiner Wand aufweist, so daß der Urin durch diese Öffnungen (2; 131) in das Innere des Urinsammlers (2a; 13) fließt, und daß die Sammeleinrichtung weiterhin einen hohlen Urinspeicher (2b; 14) umfaßt, der einstückig mit dem Urinsammler (2a; 13) verbunden ist, so daß das Innere des hohlen Urinspeichers (2b; 14) mit dem Inneren des hohlen Urinsammlers (2a; 13) in Verbindung steht, so daß der vom Urinsammler (2a; 13) gesammelte Urin weiterhin in den Urinspeicher (2a; 13) fließt, wo er gespeichert wird.

2. Urin-Sammeleinrichtung nach Anspruch 1, dadurch **gekennzeichnet,** daß die Urin-Sammelöffnungen (2) dadurch als Einschnitte gebildet sind, daß sie diagonal in die Wand des hohlen Urinsammlers (2a) eingeschnitten und die Spitzen der eingeschnittenen Teile nach außen hochgebogen sind.

3. Urin-Sammeleinrichtung nach Anspruch 1 oder 2, dadurch **gekennzeichnet,** daß ein Teil der Wand des stabartigen Körpers (1) zwischen dem Urinsammler (2a) und dem gegenüberliegenden Ende als wellenförmiger Abschnitt (3) ausgebildet ist.

4. Urin-Sammeleinrichtung nach Anspruch 3, dadurch **gekennzeichnet,** daß sie weiterhin mit einem Wasser gut absorbierenden Abdeckzylinder (4) versehen ist, der auf den stabartigen Körper (1) so aufgepaßt ist, daß er in enger Berührung mit der äußeren Oberfläche der Wand des Körpers zwischen den Urin-Sammelöffnungen (2) und dem wellenförmigen Abschnitt (3) verschiebbar ist.

5. Urin-Sammeleinrichtung nach einem der Ansprüche 1 bis 4, dadurch **gekennzeichnet,** daß sie weiterhin eine Kappe (6) umfaßt, die eng auf den Urinsammler (2a) aufsetzbar ist, um die Urin-Sammelöffnungen (2) zu verschließen.

6. Urin-Sammeleinrichtung nach Anspruch 5, dadurch **gekennzeichnet,** daß die Kappe (6) auf den stabartigen Körper (1) aufgedrückt werden kann, bis ihr offenes Ende den wellenförmigen Abschnitt (3) erreicht.

7. Urin-Sammeleinrichtung nach einem der Ansprüche 1 bis 6, dadurch **gekennzeichnet,** daß ein Testpapier (8) in das Innere des stabartigen Körpers (1) eingeführt werden kann.

8. Urin-Sammeleinrichtung nach einem der vorhergehenden Ansprüche, dadurch **gekennzeichnet,** daß sie weiterhin eine Urinspeicher-Kapsel (15) umfaßt, die eine Schneideeinrichtung (17) aufweist, um einen Teil des Urinsammlers (14) zu schneiden, sowie einen Urinbehälter (16) zur Aufnahme des aus dem Urinsammler (14) fließenden Urins, sowie einen Deckel (18), wobei der Urinsammler (14) und die Urin-Speicherkapsel (15) voneinander getrennt ausgebildet sind.

9. Urin-Sammeleinrichtung nach Anspruch 8, dadurch **gekennzeichnet,** daß der stabartige Körper (12) ein hohler, an seinen Enden geschlossener Handgriff ist, daß der Urinsammler (13) sich ausgehend vom Handgriff nach unten kegelförmig erweitert und daß der Urinsammler (14) ein zylindrischer Körper ist, der an seinem Boden durch eine leicht zu durchstoßende dünne Membran (141) verschlossen ist.

10. Urin-Sammeleinrichtung nach Anspruch 8, dadurch **gekennzeichnet,** daß die Schneideeinrichtung (17) der Urin-Speicherkapsel (15) von scharfen Klingen gebildet wird, daß der Urinbehälter (16) ein zylindrischer, am Boden geschlossener Körper ist, und daß der Deckel (18) so ausgebildet ist, daß er eng in die Öffnung (165) des Urinbehälters (16) paßt, um ein Herausfließen von Urin zu verhindern.

11. Urin-Sammeleinrichtung nach Anspruch 9 oder 10, dadurch **gekennzeichnet,** daß die Urinsammelöffnungen (131) Einschnitte oder Löcher sind.

## Revendications

1. Dispositif de prélèvement d'urine destiné à être maintenu dans l'écoulement d'urine issu d'un sujet en train d'uriner, comprenant un corps allongé similaire à une tige (1, 12) qui, à proximité de l'une de ses extrémités, est pourvue d'un collecteur d'urine creux (2a, 13) ayant une pluralité d'ouvertures de collection d'urine (2, 131) dans sa paroi, de l'urine s'écoulant à travers lesdites ouvertures (2, 131) à l'intérieur dudit collecteur d'urine (2a, 13), et comprenant en outre un accumulateur d'urine creux (2b, 14), qui est connecté de manière intégrée audit collecteur d'urine (2a, 13) de telle sorte que l'intérieur dudit accumulateur d'urine creux (2b, 14) communique avec l'intérieur dudit collecteur d'urine creux (2a, 13), de sorte que de l'urine collectée par ledit collecteur d'urine (2a, 13) s'écoule par après dans ledit accumulateur d'urine (2b, 14) où elle est stockée.

2. Dispositif de prélèvement d'urine selon la revendication 1, caractérisé en ce que les ouvertures de collection d'urine (2) sont formées sous la forme d'entailles réalisées par coupure diagonalement dans la paroi du collecteur d'urine creux (2a) et par soulèvement vers l'extérieur des extrémités des parties découpées.

3. Dispositif de prélèvement d'urine selon la revendication 1 ou 2, caractérisé en ce qu'une partie de la paroi du corps similaire à une tige (1), entre le collecteur d'urine (2a) et l'extrémité opposée, est réalisée sous la forme d'une section ondulée (3).

4. Dispositif de prélèvement d'urine selon la revendication 3, caractérisé en ce qu'il est en outre pourvu d'un cylindre de couvercle (4) hautement absorbeur d'eau adapté sur le corps similaire à une tige (1) de telle sorte à pouvoir coulisser en contact proche avec la surface externe de la paroi dudit corps, entre les ouvertures de collection d'urine (2) et la section ondulée (3).

5. Dispositif de prélèvement d'urine selon l'une des revendications 1 à 4, caractérisé en ce qu'il comprend en outre un capot (6) pouvant être adapté de manière affleurante sur ledit collecteur d'urine (2a) pour fermer les ouvertures de collection d'urine (2).

6. Dispositif de prélèvement d'urine selon la revendication 5, caractérisé en ce que le capot peut être forcé sur le corps similaire à une tige (1) jusqu'à ce que son extrémité ouverte atteigne la section ondulée (3).

7. Dispositif de prélèvement d'urine selon l'une des revendications 1 à 6, caractérisé en ce qu'un papier test (8) peut être insérée dans l'intérieur du corps similaire à une tige (1).

8. Dispositif de prélèvement d'urine selon l'une des revendications précédentes, caractérisé en ce qu'il comprend en outre une capsule de stockage d'urine (15) ayant un dispositif de coupage (17) pour couper une partie de l'accumulateur d'urine (4), un conteneur d'urine (16) pour recevoir de l'urine s'écoulant à partir de l'accumulateur d'urine (4), et un couvercle (18), l'accumulateur d'urine (4) et la capsule de stockage d'urine (15) étant réalisée de manière séparée.

9. Dispositif de prélèvement d'urine selon la revendication 8, caractérisé en ce que le corps similaire à une tige (12) est une poignée creuse fermée aux extrémités, le collecteur d'urine (13) s'évasant vers le bas à partir de la poignée sous la forme d'un cône, et l'accumulateur d'urine (14) étant un corps cylindrique fermé au fond par une fine membrane (141) facilement perçable.

10. Dispositif de prélèvement d'urine selon la revendication 8, caractérisé en ce que le dispositif de coupage (17) de la capsule de stockage d'urine (15) est réalisé sous la forme de lames coupantes, le conteneur d'urine (16) est un corps cylindrique fermé au fond, et le couvercle (18) est formé pour s'adapter de manière affleurante dans les ouvertures (165) du conteneur d'urine (16) pour empêcher les fuites d'urine à partir de celui-ci.

11. Dispositif de prélèvement d'urine selon la revendication 9 ou 10, caractérisé en ce que les ouvertures de collection d'urine (131) sont des entailles ou des trous.
